# EUROPEAN PATENT SPECIFICATION

(11) **EP 4 251 749 B1**
(45) Date of publication and mention of the grant of the patent: **07.08.2024**
(21) Application number: 21815209.8
(22) Date of filing: 25.11.2021
(51) Int. Cl.: C12N 9/78, C12N 15/62

(54) **CYTIDINE DEAMINASE VARIANTS FOR BASE EDITING**
CYTIDINDEAMINASEVARIANTEN ZUR BASENEDITIERUNG
VARIANTS DE LA CYTIDINE DÉSAMINASE POUR L'ÉDITION DE BASES

(30) Priority: 27.11.2020 IT 202000028688
(43) Date of publication of application: 04.10.2023
(73) Proprietor: Consiglio Nazionale Delle Ricerche, 00185 Roma (IT)
(72) Inventor: CONTICELLO, Silvestro, 50134 Firenze (IT); DONATI, Francesco, 52100 Arezzo (IT); SEVERI, Francesco, 59100 Prato (IT)
(74) Representative: Leone, Mario
(86) International application number: PCT/EP2021/082971
(87) International publication number: WO 2022/112404

(56) References cited:
- WO-A1-2019/023680
- WO-A2-2020/077138

## Description

### Background of the invention

The present invention relates to the field of biotechnology, and in particular to medical biotechnology, since it concerns base editors for genomic editing capable of inducing specific point mutations that can be used with a high level of safety as they reduce unwanted mutations.

### Prior Art

Base editors are chimeric molecules in which a domain, usually Cas9, which is able to bind DNA in a specific way via guide RNA is fused with a deaminase that induces modification of a specific base, used in genomic editing techniques. These tools make it possible to act on specific sites in the human genome by inducing deletions and insertions in a controlled manner. These molecules allow the modification of single bases in a specific region of DNA without inducing a break in the double chain. For cytosine to thymine mutation (C>T editing), the most commonly used deaminase is rat APOBEC1, a potent DNA mutator that acts physiologically on RNA.

It is known that cases of off-target mutation (off-target effects) can occur, although the specificity of Cas9 is controlled by a 20 bp sequence present on a guide RNA (gRNA) and by the presence of a specific sequence (PAM). For example, deletions and recombinations may occur on genomic sites other than those planned due to a misalignment of the gRNA in regions with 3-5 possible bases other than those of the gRNA (Hsu, P. D. et al., 2013, DNA targeting specificity of RNA-guided Cas9 nucleases. Nature Biotechnology, 31(9), 827-832.; Fu, Y. et al., 2013, High-frequency off-target mutagenesis induced by CRISPR-Cas nucleases in human cells. Nature Biotechnology, 31(9), 822-826). Or off-target mutations can occur, both on DNA and on RNA, due to deaminase over-expression, e.g. base editors are known to induce mutations in mouse embryos (Lee, H. K., Smith, H. E., Liu, C., Willi, M., & Hennighausen, L., 2020, Cytosine base editor 4 but not adenine base editor generates off-target mutations in mouse embryos. Commun Biol, 3(1), 19. doi:10.1038/s42003-019-0745-3), in human cells (McGrath, E., Shin, H., Zhang, L., Phue, J.-N., Wu, W. W., Shen, R.-F., ... Ye, Z., 2019, Targeting specificity of APOBEC-based cytosine base editor in human iPSCs determined by whole genome sequencing. Nat Commun, 10(1), 5353. doi:10.1038/s41467-019-13342-8) and in rice (Jin, S., Zong, Y., Gao, Q., Zhu, Z., Wang, Y., Qin, P., ... Gao, C., 2019, Cytosine, but not adenine, base editors induce genome-wide off-target mutations in rice. Science, 364(6437), 292-295. doi:10.1126/science.aaw7166). This occurs in both coding and noncoding sequences, generating missense (wrong sense), nonsense (with no sense) mutations, such as splice sites (cut and paste sites) and at the 5' / 3' UTR (Grünewald, J., Zhou, R., Garcia, S. P., Iyer, S., Lareau, C. A., Aryee, M. J., & Joung, J. K., 2019, Transcriptome-wide off-target RNA editing induced by CRISPR-guided DNA base editors. Nature, 569(7756), 433-437; Zhou, C., Sun, Y., Yan, R., Liu, Y., Zuo, E., Gu, C., ... Yang, H., 2019, Off-target RNA mutation induced by DNA base editing and its elimination by mutagenesis. Nature, 571(7764), 275-278. doi:10.1038/s41586-019-1314-0). To reduce this effect, the replacement of the APOBEC1 component in BE3 with rat APOBEC1 mutants has been described, characterised by reduced RNA editing activity and based on previously analysed mutations (Yamanaka, S., Poksay, K. S., Balestra, M. E., Zeng, G. Q., & Innerarity, T. L.,1994, Cloning and mutagenesis of the rabbit apoB mRNA editing protein. A zinc motif is essential for catalytic activity, and noncatalytic auxiliary factor(s) of the editing complex are widely distributed. Journal of Biological Chemistry, 269(34), 21725-21734; Navaratnam, N., Bhattacharya, S., Fujino, T., Patel, D., Jarmuz, A. L., & Scott, J., 1995, Evolutionary origins of apoB mRNA editing: Catalysis by a cytidine deaminase that has acquired a novel RNA-binding motif at its active site. Cell, 81(2), 187-195. MacGinnitie, A. J., Anant, S., & Davidson, N. O., 1995. Mutagenesis of apobec-1, the catalytic subunit of the mammalian apolipoprotein B mRNA editing enzyme, reveals distinct domains that mediate cytosine nucleoside deaminase, RNA binding, and RNA editing activity. Journal of Biological Chemistry, 270(24), 14768-14775; Teng, B. B., Ochsner, S., Zhang, Q., Soman, K. V., Lau, P. P., & Chan, L., 1999, Mutational analysis of apolipoprotein B mRNA editing enzyme (APOBEC1): Structure-function relationships of RNA editing and dimerization. Journal of Lipid Research, 40(4), 623-635; Chen, Z., Eggerman, T. L., Bocharov, A. V., Baranova, I. N., Vishnyakova, T. G., Csako, G., & Patterson, A. P., 2010, Hypermutation induced by APOBEC-1 overexpression can be eliminated. RNA, 16(5), 1040-1052).

Over the years Cas9 with a higher target specificity have been developed (Hu et al 2018; Casini et al 2018) and APOBEC1 mutants have been identified that induce fewer off-target effects (Doman, J. L., Raguram, A., Newby, G. A., & Liu, D. R.,2020, Evaluation and minimization of Cas 9-independent off-target DNA editing by cytosine base editors. Nat Biotechnol, 38(5), 620-628. doi:10.1038/s41587-020-0414-6 Zuo; E., Sun, Y., Wei, W., Yuan, T., Ying, W., Sun, H., ... Yang, H., 2019, Cytosine base editor generates substantial off-target single-nucleotide variants in mouse embryos. Science, 364(6437), 289-292; Yu, Y., Leete, T. C., Born, D. A., Young, L., Barrera, L. A., Lee, S.-J., Gaudelli, N. M., 2020, Cytosine base editors with minimized unguided DNA and RNA off-target events and high on-target activity. Nat Commun, 11(1), 2052. doi:10.1038/s41467-020-15887-5).

The US patent application, publication number US2018312828, describes fusion proteins for gene editing in which the deaminase activity is carried out by the enzyme APOBEC1 and its mutants such as those with mutants at position 132 in which the substitution can be made with any amino acid.

The international patent application, publication number WO2019/241649, describes variants of cytidine deaminase prepared using the PACE (Phage Assisted Continuous Evolution) technique as well as vectors incorporating them with the Cas9 domain. The APOBEC1 variants described are characterised by mutations at positions R33, G45, N57, N65, Y75, T101, F113, F113, A123, S149, A165, H166, T204, F205, and W224.

The international patent application, publication number WO2019042284 describes base editors containing a catalytically inactive version of the bacterium Lachnospiraceae Cpf1 (LbCpf1) and cytidine deaminase, such as for example the APOBEC protein in various forms such as for example APOBEC1 with mutation W90Y or R126E, or APOBEC3A with mutations W104A, Y130F, D131Y, D31E and combinations thereof.

The international patent application, publication number WO2020/077138, describes the cytidine deaminase APOBEC1 having mutations in the amino acid positions P29, R33, K34, E181 and/or L182 that decrease its RNA editing activity while preserving its DNA editing activity.

### Technical problem

The use of base editors is an efficient tool for genomic editing, but their expression can often lead to unwanted mutations in both RNA and DNA.

The problem of developing systems that reduce or eliminate this drawback is therefore strongly felt in the field of the present invention.

In the light of what is known in the state of the art, the inventors of the present invention have developed, by means of bacterial screenings and tests in human cells, mutants of the deaminase APOBEC1 whose use within the base editor induces a considerably reduced number of mutations both on RNA and on DNA and therefore can be used to induce genomic editing with a higher level of safety.

### Object of the invention

The technical problem is therefore solved by providing variant nucleotide sequences of the wild-type protein cytidine deaminase APOBEC1 of sequence
SEQ. ID. NO.1

ATGAGTTCCGAGACAGGCCCTGTAGCTGTTGATCCCACTCTGAGGAGAAGAATTGAGCCCCACGAG
TTTGAAGTCTTCTTTGACCCCCGGGAACTTCGGAAAGAGACCTGTCTGCTGTATGAGATCAACTGG
GGAGGAAGGCACAGCATCTGGCGACACACGAGCCAAAACACCAACAAACACGTTGAAGTCAATTTC
ATAGAAAAATTTACTACAGAAAGATACTTTTGTCCAAACACCAGATGCTCCATTACCTGGTTCCTG
TCCTGGAGTCCCTGTGGGGAGTGCTCCAGGGCCATTACAGAATTTTTGAGCCGATACCCCCATGTA
ACTCTGTTTATTTATATAGCACGGCTTTATCACCACGCAGATCCTCGAAATCGGCAAGGACTCAGG
GACCTTATTAGCAGCGGTGTTACTATCCAGATCATGACGGAGCAAGAGTCTGGCTACTGCTGGAGG

AATTTTGTCAACTACTCCCCTTCGAATGAAGCTCATTGGCCAAGGTACCCCCATCTGTGGGTGAGG
CTGTACGTACTGGAACTCTACTGCATCATTTTAGGACTTCCACCCTGTTTAAATATTTTAAGAAGA
AAACAACCTCAACTCACGTTTTTCACGATTGCTCTTCAAAGCTGCCATTACCAAAGGCTACCACCC
CACATCCTGTGGGCCACAGGGTTGAAATGA

The object of the present invention is a nucleotide sequence coding for a variant of the cytidine deaminase protein chosen from the group consisting of
sequence SEQ.ID.NO. 2 [F66L (C196T) /V179I (G535A)] 
ATGAGTTCCGAGACAGGCCCTGTAGCTGTTGATCCCACTCTGAGGAGAAGAATTGAGCCCCACGAG
TTTGAAGTCTTCTTTGACCCCCGGGAACTTCGGAAAGAGACCTGTCTGCTGTATGAGATCAACTGG
GGAGGAAGGCACAGCATCTGGCGACACACGAGCCAAAACACCAACAAACACGTTGAAGTCAATCTC
ATAGAAAAATTTACTACAGAAAGATACTTTTGTCCAAACACCAGATGCTCCATTACCTGGTTCCTG
TCCTGGAGTCCCTGTGGGGAGTGCTCCAGGGCCATTACAGAATTTTTGAGCCGATACCCCCATGTA
ACTCTGTTTATTTATATAGCACGGCTTTATCACCACGCAGATCCTCGAAATCGGCAAGGACTCAGG
GACCTTATTAGCAGCGGTGTTACTATCCAGATCATGACGGAGCAAGAGTCTGGCTACTGCTGGAGG
AATTTTGTCAACTACTCCCCCTCGAATGAAGCTCATTGGCCAAGGTACCCCCATCTGTGGGTGAGG
CTGTACATACTGGAACTCTACTGCATCATTTTAGGACTTCCACCCTGTTTAAATATTTTAAGAAGA
AAACAACCTCAACTCACGTTTTTCACGATTGCTCTTCAAAGCTGCCATTACCAAAGGCTACCACCC
CACATCCTGTGGGCCACAGGGTTGAAA
SEQ.ID.NO.3 [I195N T584A] 
ATGAGTTCCGAGACAGGCCCTGTAGCTGTTGATCCCACTCTGAGGAGAAGAATTGAGCCCCACGAG
TTTGAAGTCTTCTTTGACCCCCGGGAACTTCGGAAAGAGACCTGTCTGCTGTATGAGATCAACTGG
GGAGGAAGGCACAGCATCTGGCGACACACGAGCCAAAACACCAACAAACACGTTGAAGTCAATTTC
ATAGAAAAATTTACTACAGAAAGATACTTTTGTCCAAACACCAGATGCTCCATTACCTGGTTCCTG
TCCTGGAGTCCCTGTGGGGAGTGCTCCAGGGCCATTACAGAATTTTTGAGCCGATACCCCCATGTA
ACTCTGTTTATTTATATAGCACGGCTTTATCACCACGCAGATCCTCGAAATCGGCAAGGACTCAGG
GACCTTATTAGCAGCGGTGTTACTATCCAGATCATGACGGAGCAAGAGTCTGGCTACTGCTGGAGG
AATTTTGTCAACTACTCCCCTTCGAATGAAGCTCATTGGCCAAGGTACCCCCATCTGTGGGTGAGG
CTGTACGTACTGGAACTCTACTGCATCATTTTAGGACTTCCACCCTGTTTAAATAATTTAAGAAGA
AAACAACCTCAACTCACGTTTTTCACGATTGCTCTTCAAAGCTGCCATTACCAAAGGCTACCACCC
CACATCCTGTGGGCCACAGGGTTGAAA
SEQ.ID.NO.4 [C82R (T244C)]

ATGAGTTCCGAGACAGGCCCTGTAGCTGTTGATCCCACTCTGAGGAGAAGAATTGAGCCCCACGAG
TTTGAAGTCTTCTTTGACCCCCGGGAACTTCGGAAAGAGACCTGTCTGCTGTATGAGATCAACTGG
GGAGGAAGGCACAGCATCTGGCGACACACGAGCCAAAACACCAACAAACACGTTGAAGTCAATtTC

ATAGAAAAATTTACTACAGAAAGATACTTTTGTCCAAACACCAGACGCTCCATTACCTGGTTCCTG
TCCTGGAGTCCCTGTGGGGAGTGCTCCAGGGCCATTACAGAATTTTTGAGCCGATACCCCCATGTA
ACTCTGTTTATTTATATAGCACGGCTTTATCACCACGCAGATCCTCGAAATCGGCAAGGACTCAGG
GACCTTATTAGCAGCGGTGTTACTATCCAGATCATGACGGAGCAAGAGTCTGGCTACTGCTGGAGG
AATTTTGTCAACTACTCCCCtTCGAATGAAGCTCATTGGCCAAGGTACCCCCATCTGTGGGTGAGG
CTGTACGTACTGGAACTCTACTGCATCATTTTAGGACTTCCACCCTGTTTAAATATTTTAAGAAGA
AAACAACCTCAACTCACGTTTTTCACGATTGCTCTTCAAAGCTGCCATTACCAAAGGCTACCACCC
CACATCCTGTGGGCCACAGGGTTGAAATGT

A further object of the present invention are the variants of the cytidine deaminase protein encoded by the nucleotide sequence selected in the group consisting of SEQs. ID. NO.2, SEQ. ID. NO.3, SEQ. ID. NO.4.

The mRNAs transcribed by any of the nucleotide sequences of sequence SEQ are also an object of the present invention. ID. NO.2, SEQ. ID. NO.3, SEQ. ID. NO.4 and each of their splicing variants containing at least one of the mutations F66L +V179I, I195N, C82R.

A further object of the present invention is any vector and/or host cell comprising a nucleotide sequence chosen from the group consisting of SEQ. ID. NO.2, SEQ. ID. NO.3, SEQ. ID. NO.4 or the transcribed mRNA a nucleotide sequence chosen from the group consisting of SEQ. ID. NO.2, SEQ. ID. NO.3, SEQ. ID. NO.4, and each of its splicing variants.

Another object of the present invention is a fusion protein comprising:
the variant of the cytidine deaminase protein encoded by a nucleotide sequence chosen from the group consisting of SEQ. ID. NO.2, SEQ. ID. NO.3, SEQ. ID. NO.4.
and a protein capable of carrying APOBEC1 mutants on nucleic acids or its functional domain.

A further object of the present invention is a coding sequence for a fusion protein comprising:
a nucleotide sequence chosen from the group consisting of SEQ. ID. NO.2, SEQ. ID. NO.3, SEQ. ID. NO.4, and
a sequence coding for a protein capable of carrying APOBEC1 mutants on nucleic acids or its functional domain.

Any vector and/or host cell comprising a sequence coding for a fusion protein is also an object of the present invention.

Another object of the present invention is the use of the fusion protein as a base editor.

A further object of the present invention is a base editing process comprising the use of the base editor constituted by the fusion protein.

A further object of the present invention is a kit comprising all the elements necessary to carry out a base editing process comprising the use of the base editor constituted by the fusion protein.

Further features of the present invention will be evident and clear from the following detailed description with reference to experimental data presented in the present description and the attached figures.

### Brief description of the figures

Figure 1 shows the editing percentages of the different base editors on the selected genomic targets; each graph represents the editing percentages of each cytosine within the editing window of a target gene; EMX1 has two editable Cs, while HEK#3 has three editable Cs; within each graph the base preceding the edited C is shown; a Base Editor without APOBEC1 (woA1) and with the catalytically inactive mutant E63A were used as negative controls; BE3 (A1) was used as a reference for the editing capacity of RNA editing-deficient mutants.
Figure 2 shows the RNA editing frequency for the different base editors, the analysis was performed in duplicate, BE3 (BE-A1) induces a high editing frequency over the whole transcriptome, Base Editors with mutants induce an RNA editing frequency comparable to the negative controls BE3woA1 and BE-E63A.
Figure 3 shows the LOGO plot of nucleotides surrounding the modified site within all sequenced samples, no enrichment is present with the exception of the sample with Base Editor over-expression with wt APOBEC1 (BE-A1).
Figure 4 shows the number of unique SNVs present on transcripts from both DNA strands; only the Base Editor with wt APOBEC1 (A1) induces off-target mutations.

### Detailed description of the invention

### Definitions

Within the meaning of the present invention, APOBEC1 is the catalytic polypeptide 1 of the enzyme cytidine deaminase that converts cytosine to uracil in the messenger RNA (mRNA) of apolipoprotein B.

Within the meaning of the present invention, chimeric protein or fusion protein means any protein experimentally created by the translation of at least two genes joined together and originally coding for separate proteins.

Within the meaning of the present invention, base editor means a fusion protein comprising a variant of the enzyme cytidine deaminase and a protein capable of carrying mutants of APOBEC1 on nucleic acids or functional domain thereof.

Within the meaning of the present invention, a protein capable of carrying APOBEC1 mutants or functional domain thereof is any protein or functional domain capable of using a guide RNA to carry APOBEC1 mutants on the region complementary to the guide RNA. Proteins or domains capable of performing said function are also proteins from the CRISPR-associated protein (Cas) family, where CRISPR means CRISPR (Clustered Regularly Interspaced Short Palindromic Repeats).

Proteins capable of carrying APOBEC1 mutants, associated with genomic editing, can be chosen from the group consisting of: Zinc-Finger Nucleases (ZFNs), Transcription activator-like effector nucleases (TALENs), Meganuclease, Cas9 and its laboratory-generated variants, Cas12a and its lab-created variants, SpCas9 type, VRER SpCas9, VQR SpCas9, EQR SpCas9, xCas9-3.7, eSpCas9 (1.0), eSpCas9 (1.1), Cas9-HF1, HypaCas9, evoCas9, HiFi Cas9, ScCas9, StCas9, NmCas9, SaCas9, CiCas9, CasX), and Cascade/Cas3 and its lab-created variants.

Within the meaning of the present invention, base editing is the process of producing point mutations in the RNA without breaking the molecule.

Within the meaning of the present invention, Cas9 means CRISPR 9 (CRISPR associated protein 9), i.e. a DNA endonuclease enzyme with guide RNA associated with the CRISPR adaptive immune system produced by Streptococcus pyogenes and other bacteria.

Within the meaning of the present invention, vector means any system capable of transporting exogenous DNA sequences into a host cell and allowing them to be integrated into the host genome and the expression thereof in the host cell. The vector may be of plasmid origin.

Within the meaning of the present invention, host cell means the target cell into which the exogenous genome is inserted and expressed.

Within the meaning of the present invention, splicing (assembly) is understood to be the process of modifying transcribed mRNA by which intronic sequences are removed and exons are joined together, whereby splicing variants are understood to be all sequences, even intermediate sequences, which are produced during the splicing process.

Since information is only available on the catalytic site of APOBEC1 but there is no precise information on the regions of APOBEC1 involved in the recognition and activity of APOBEC1 on DNA and RNA, the inventors of the present invention have first investigated and then verified that the C-terminal portion of APOBEC-1 has a function in RNA editing activity and in particular that deletion of the 40 amino acid C-terminal domain inhibits the enzymatic activity of APOBEC1 on target RNA.

A set of APOBEC1 mutants was selected by two specific bacterial screens starting from a specific set of mutants generated by mutagenic PCR. A set of APOBEC1 mutants characterised by a reduced RNA editing capacity but capable of maintaining DNA editing capacity was therefore selected by coupling two screening methods, one to select mutants unable to edit RNA and another to select mutants retaining the ability to edit DNA.

In view of the foregoing, an object of the invention is a nucleotide sequence coding for a variant of the cytidine deaminase protein chosen from the group consisting of:
sequence SEQ.ID.NO. 2, containing the point mutations F66L and V179I; sequence SEQ.ID.NO.3 containing the I195N mutation;
sequence SEQ.ID.NO.4 containing the C82R mutation.

A further object of the present invention are the variants of the cytidine deaminase protein encoded by a nucleotide sequence chosen from the group consisting of sEQ nucleotide sequence. ID. NO.2, sEQ nucleotide sequence. ID. NO.3, SEQ nucleotide sequence. ID. NO.4.

Another object of the present invention is the mRNA transcribed by a nucleotide sequence chosen from the group consisting of: SEQ. ID. NO.2, SEQ. ID. NO.3, SEQ. ID. NO.4 and any splicing variant that retains at least one of the F66L+V179I, I195N, C82R mutations.

A further object of the present invention is any vector and/or host cell comprising a nucleotide sequence chosen from the group consisting of SEQ. ID. NO.2, SEQ. ID. NO.3, SEQ. ID. NO.4 or RNA transcribed by them or corresponding cDNA.

Another object of the present invention is a fusion protein comprising:
the variant of the cytidine deaminase protein encoded by the nucleotide sequence selected from the group consisting of SEQ. ID. NO.2, SEQ. ID. NO.3, SEQ. ID. NO.4.
and a protein capable of carrying APOBEC1 mutants on nucleic acids or its functional domain.

As well as the coding sequence for said fusion protein and any vector and/or host cell comprising the coding sequence for a fusion protein.

Preferably the protein capable of carrying APOBEC1 mutants, associated with genomic editing, is chosen from the group consisting of: Zinc-Finger Nucleases (ZFNs), Transcription activator-like effector nucleases (TALENs), Meganucleases, Cas9 and its lab-created variants, Cas12a and its lab-created variants, type SpCas9, VRER SpCas9, VQR SpCas9, EQR SpCas9, xCas9-3.7, eSpCas9 (1.0), eSpCas9 (1.1), Cas9-HF1, HypaCas9, evoCas9, HiFi Cas9, ScCas9, StCas9, NmCas9, SaCas9, CiCas9, CasX), and Cascade/Cas3 and its lab-created variants.

More preferably it is Cas9.

Preferably the vector is a plasmid vector.

Preferably the plasmid is chosen from the group consisting of: pAIDExpressPuro, pEGFP-C3, BE3, pFLAG.

Another object of the present invention is the use of the fusion protein as a base editor.

A further object of the present invention is a base editing process comprising the use of the base editor constituted by the fusion protein.

Preferably the process of base editing comprises the following stages:
a) cell culture
b) transfection of cells by means of a plasmid vector comprising a sequence coding for the fusion protein, a sequence coding for the guide RNA and at least one gene for the selection with antibiotic
c) selection of cells that have been transfected with antibiotic until a selected cell culture with the desired genomic mutation is obtained.

A further object of the present invention is a kit which comprises all the elements necessary to carry out a base editing process which comprises the use of the base editor constituted by the fusion protein and in particular comprising at least one plasmid comprising a coding sequence for the fusion protein a sequence coding for the guide RNA and at least one gene for the selection with antibiotic.

### Examples

### Materials and Methods

Cell culture: the cell line, commercially available from the ATCC American Type Culture Collection, HEK293T from human embryonic kidney was used. Tissue culture medium was prepared from Dulbecco's Modified Eagle's Medium (DMEM) (Euroclone). DMEM was supplemented with 10% foetal bovine serum (FBS) and 1% penicillin/streptomycin sulphate, 1% L-glutamine. Cells were cultured in an incubator at 37°C in 5% CO₂. When the cells were confluent, they were divided into new plates containing fresh medium. Cell counting was performed in a counting chamber of the Neubauer haemocytometer.

Transfection: transient transfections of HEK293T cells were performed using polyethylenimine (PEI) following the manufacturer's instructions. Transfection of 1 µg of plasmid DNA was performed by incubating the DNA with 3 µl of PEI [1 µg/µl] in 200 ul of DMEM. After 15 minutes of incubation, the total volume was transferred drop by drop into the 2 ml volume of the cell culture, in this case a 6-well plate. The same method was enlarged or reduced to allow transfection of higher or lower amounts of DNA, while maintaining the same ratio between DNA and PEI and between the volume of DMEM and the volume of cell culture medium.

Flow cytometry: Approximately 2-5 x 105 cells were transferred into 5 ml polystyrene round-bottom tubes, centrifuged at 300xg at 4°C, washed once in PBS and centrifuged again. The cell pellet was resuspended in 300 µl PBS and analysed. The cytofluorometers used were a FlowCytometer BD Accuri C6 and a Beckaman Coulter CytoFLEX.

DNA and RNA extraction: DNA from eukaryotic cells was extracted with the guided genomic purification kit (Promega) following the manufacturer's instructions and quantified with the Epoch BioTek spectrophotometer.

RNA was extracted from the cells using Trizol reagent (Life Technologies) and then purified from potential DNA contamination using the recombinant DNAse I kit (Roche) following the manufacturer's instructions. Retro-transcription was performed with the high-capacity cDNA reverse transcription kit (Applied Biosystem).

Construction of vectors and plasmids: plasmids were cloned starting from pre-existing vectors present in the laboratory or obtained from Addgene. The insertion of small DNA fragments (<100 bp) was performed by synthesising two complementary single-stranded oligonucleotides, which were then inserted into specific restriction sites after annealing (denaturation) and phosphorylation, following the "ZhangLab General Cloning Protocol" (Addgene link). The insertion of larger DNA fragments was performed by PCR amplification of the chosen sequence with High-Fidelity polymerase and the use of 5'-extended primers (initiators) containing a restriction site. PCR amplification of DNA fragments to be cloned or sequenced was performed using KOD Hot Start DNA Polymerase (Novagen).

APOBEC1 mutants were selected through two bacterial screens starting from a pool of mutants generated by mutagenic PCR. Screening of APOBEC1 mutants with reduced RNA editing capacity was performed by adapting the use of the arabinose (Ara) operon to induce cell death. The ccdB gene, a topoisomerase poison, was placed under the control of the Ara promoter. The F15L mutation (T>C) was inserted in the AraC gene, the regulator of the Ara operon, in order to make the operon insensitive to the addition of arabinose. The mutation was designed to be a possible target for editing APOBEC1 on RNA. In the case of RNA editing at this site, cytosine is edited into uracil and the wild-type sequence is restored. The addition of arabinose induces the expression of ccdB in bacteria where RNA editing has taken place and this in turn induces cell death. In this way, only bacteria that express an APOBEC1 mutant that does not edit RNA survive, permitting the selection of APOBEC1 mutants that are unable to edit RNA. A rifampicin antibiotic resistance assay was used to select APOBEC1 mutants that retain the ability to edit DNA, where bacteria are unable to grow in the presence of rifampicin unless they acquire mutations on the RNA polymerase that allow it to function even in the presence of the antibiotic. The presence of APOBEC1 mutants that induce a mutator phenotype facilitates the introduction of mutations that allow the bacteria to grow. The combination of the two screening methods made it possible to select mutants that retain the ability to edit DNA without the ability to edit RNA.

The following mutants were initially selected: F66L+V179I; I195N; C82R; L182H; R132G.

At the end of the experiment, the sequences F66L+V179I, I195N, C82R were further selected.

DNA sequencing of the edited genomic targets was performed using amplicon target sequencing using Illumina MiSeq technology. Genomic targets were amplified by PCR. Amplicons were prepared for sequencing using Illumina's TruSeq DNA Nano Kit, pooled into libraries and sequenced with MiSeq Reagent Kit v2 micro, 2 x 150 cycles.

APOBEC1 mutants were fused with Cas9 as in the original BE3 construct described in Komor AC, YB, K., MS, P., JA, Z., & DR., L. (2016). Programmable editing of a target base in genomic DNA without double-stranded DNA cleavage. Nature, 533(7603), 420-424. The efficiency of mutants on five genomic targets was evaluated to test base editors as described in Komor AC, YB, K., MS, P., JA, Z., & DR., L. (2016). Programmable editing of a target base in genomic DNA without double-stranded DNA cleavage. Nature, 533(7603), 420-424.

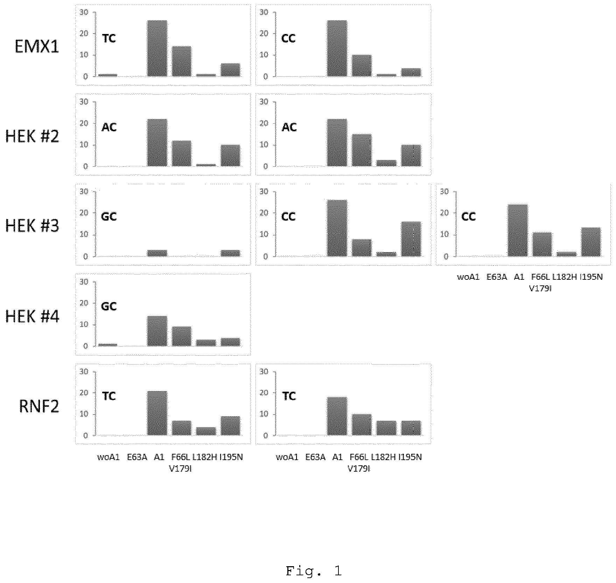

HEK293T cells were transfected with base editors and sgRNAs specific for these targets. In order to assess editing levels, genomic DNA was extracted from transfected cells after 72 hours and DNA fragments enclosing the target editing site were amplified. Plasmids lacking APOBEC1 or containing the catalytically inactive mutant E63A were used as negative controls. MiSeq Illumina sequencing was performed on all samples. The results of the sequencing run, in Figure 1, show that BE3 (the original base editor, denoted as A1) is the most efficient, as it is able to edit all cytosines present within the target sequences (14-26%). However, the mutants are also able to induce C to T conversions on all cytosines (4-16%), although less efficiently than BE3, particularly the RA7 mutant (1-7%). The base editors obtained by means of the mutants of the present invention have retained the ability to modify cytosines.

After demonstrating that base editors with APOBEC1 mutants are able to edit several genomic targets, it was assessed whether the mutants, unlike BE3, do not cause C to U RNA editing. To this end, mRNA sequencing was performed with Illumina NextSeq technology on RNA samples obtained from cells transfected with Base Editor directed towards the genomic target RNF2. The target RNF2 was chosen because this sgRNA induces very low levels of off-target DNA mediated by sgRNA mistargeting. Therefore, the only expected off-target mutations derive only from deaminase mistargeting, both on RNA and DNA.

The L182H mutant, similar to that described in Grünewald, J., Zhou, R., Garcia, S. P., Iyer, S., Lareau, C. A., Aryee, M. J., & Joung, J. K. (2019), was also identified. Transcriptome-wide off-target RNA editing induced by CRISPR-guided DNA base editors. Nature, 569(7756), 433-437, named L182A, but with lower off-target levels when compared to the negative control.

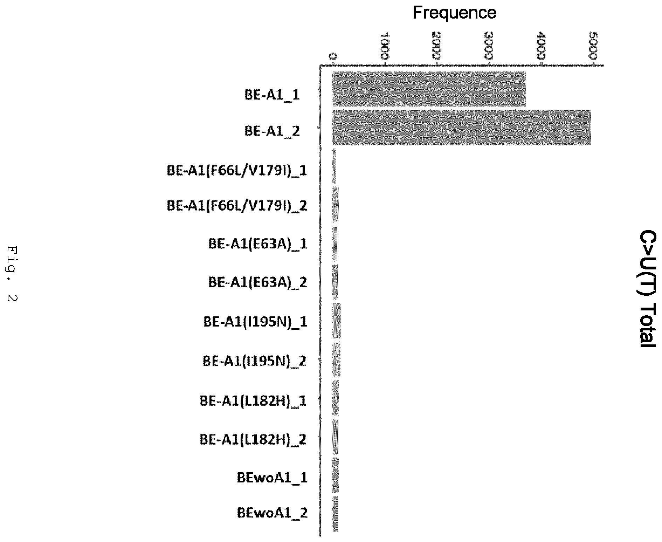

The results in Figure 2 show that the selected base editors induced low levels of RNA editing compared to the original BE3 (BE-A1), levels comparable to those of controls lacking deaminating enzyme activity (BE3woA1 and BE-E63A), the base editors were analysed in duplicate.

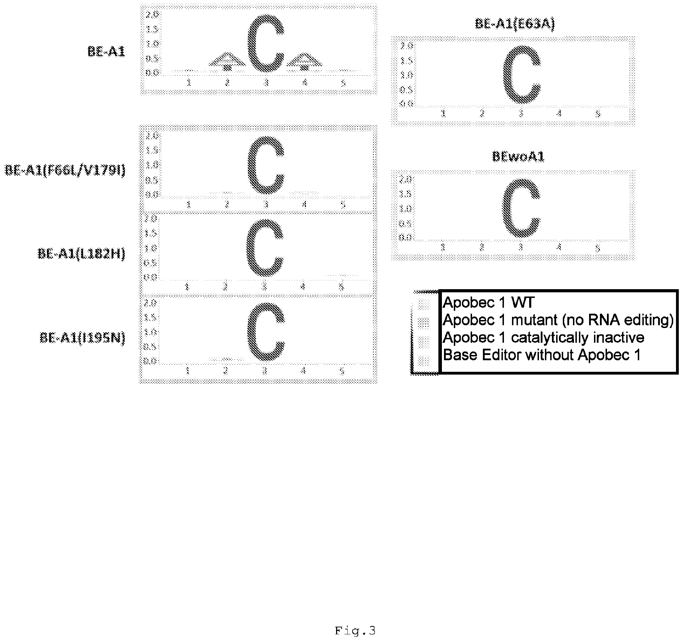

The few mutations found in the samples with APOBEC1 mutants and negative controls are probably due to random mutations, in fact, the analysis of the context of the sequence surrounding the editing sites, shown in figure 3, shows that while APOBEC1 is known to preferentially deaminate cytosines preceded by adenines/thymines, no such enrichment can be observed for APOBEC1 mutants.

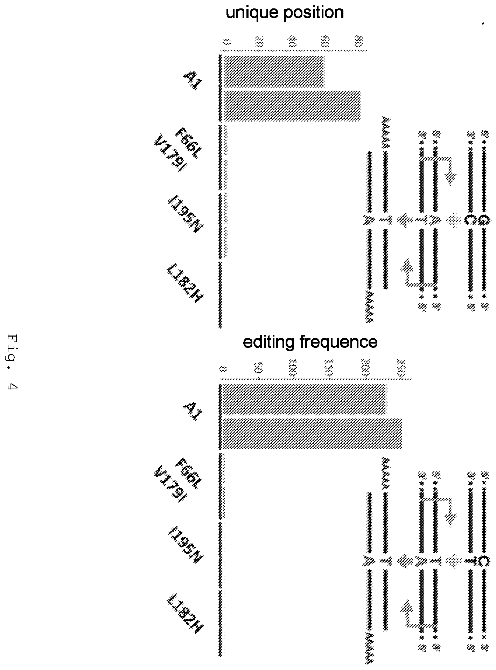

Furthermore, since single nucleotide variants occurring on the same genomic site but on transcripts originating from the two opposite DNA strands cannot be attributed to RNA modification, it was possible to assess the presence of off-target mutations on DNA using transcriptomic data. While off-target mutations were present in the samples in which BE3 was overexpressed, very few mutations were present in samples expressing the APOBEC1 mutants, as shown in Figure 4.

These results indicate that APOBEC1 mutants can be used for genomic editing with very low levels of off-target effects.

## Claims

1. Polynucleotide coding for a variant of the cytidine deaminase protein chosen from the group consisting of:
SEQ.ID.NO. 2 
ATGAGTTCCGAGACAGGCCCTGTAGCTGTTGATCCCACTCTGAGGAGAAGAATTGAGCCCC
ACGAGTTTGAAGTCTTCTTTGACCCCCGGGAACTTCGGAAAGAGACCTGTCTGCTGTATGA
GATCAACTGGGGAGGAAGGCACAGCATCTGGCGACACACGAGCCAAAACACCAACAAACAC
GTTGAAGTCAATCTCATAGAAAAATTTACTACAGAAAGATACTTTTGTCCAAACACCAGAT
GCTCCATTACCTGGTTCCTGTCCTGGAGTCCCTGTGGGGAGTGCTCCAGGGCCATTACAGA
ATTTTTGAGCCGATACCCCCATGTAACTCTGTTTATTTATATAGCACGGCTTTATCACCAC
GCAGATCCTCGAAATCGGCAAGGACTCAGGGACCTTATTAGCAGCGGTGTTACTATCCAGA
TCATGACGGAGCAAGAGTCTGGCTACTGCTGGAGGAATTTTGTCAACTACTCCCCCTCGAA
TGAAGCTCATTGGCCAAGGTACCCCCATCTGTGGGTGAGGCTGTACATACTGGAACTCTAC
TGCATCATTTTAGGACTTCCACCCTGTTTAAATATTTTAAGAAGAAAACAACCTCAACTCA
CGTTTTTCACGATTGCTCTTCAAAGCTGCCATTACCAAAGGCTACCACCCCACATCCTGTG
GGCCACAGGGTTGAAA
SEQ.ID.NO.3 
ATGAGTTCCGAGACAGGCCCTGTAGCTGTTGATCCCACTCTGAGGAGAAGAATTGAGCCCC
ACGAGTTTGAAGTCTTCTTTGACCCCCGGGAACTTCGGAAAGAGACCTGTCTGCTGTATGA
GATCAACTGGGGAGGAAGGCACAGCATCTGGCGACACACGAGCCAAAACACCAACAAACAC
GTTGAAGTCAATTTCATAGAAAAATTTACTACAGAAAGATACTTTTGTCCAAACACCAGAT
GCTCCATTACCTGGTTCCTGTCCTGGAGTCCCTGTGGGGAGTGCTCCAGGGCCATTACAGA
ATTTTTGAGCCGATACCCCCATGTAACTCTGTTTATTTATATAGCACGGCTTTATCACCAC
GCAGATCCTCGAAATCGGCAAGGACTCAGGGACCTTATTAGCAGCGGTGTTACTATCCAGA
TCATGACGGAGCAAGAGTCTGGCTACTGCTGGAGGAATTTTGTCAACTACTCCCCTTCGAA
TGAAGCTCATTGGCCAAGGTACCCCCATCTGTGGGTGAGGCTGTACGTACTGGAACTCTAC
TGCATCATTTTAGGACTTCCACCCTGTTTAAATAATTTAAGAAGAAAACAACCTCAACTCA
CGTTTTTCACGATTGCTCTTCAAAGCTGCCATTACCAAAGGCTACCACCCCACATCCTGTG
GGCCACAGGGTTGAAA
SEQ.ID.NO.4

ATGAGTTCCGAGACAGGCCCTGTAGCTGTTGATCCCACTCTGAGGAGAAGAATTGAGCCCC
ACGAGTTTGAAGTCTTCTTTGACCCCCGGGAACTTCGGAAAGAGACCTGTCTGCTGTATGA
GATCAACTGGGGAGGAAGGCACAGCATCTGGCGACACACGAGCCAAAACACCAACAAACAC
GTTGAAGTCAATtTCATAGAAAAATTTACTACAGAAAGATACTTTTGTCCAAACACCAGAC
GCTCCATTACCTGGTTCCTGTCCTGGAGTCCCTGTGGGGAGTGCTCCAGGGCCATTACAGA
ATTTTTGAGCCGATACCCCCATGTAACTCTGTTTATTTATATAGCACGGCTTTATCACCAC

GCAGATCCTCGAAATCGGCAAGGACTCAGGGACCTTATTAGCAGCGGTGTTACTATCCAGA
TCATGACGGAGCAAGAGTCTGGCTACTGCTGGAGGAATTTTGTCAACTACTCCCCtTCGAA
TGAAGCTCATTGGCCAAGGTACCCCCATCTGTGGGTGAGGCTGTACGTACTGGAACTCTAC
TGCATCATTTTAGGACTTCCACCCTGTTTAAATATTTTAAGAAGAAAACAACCTCAACTCA
CGTTTTTCACGATTGCTCTTCAAAGCTGCCATTACCAAAGGCTACCACCCCACATCCTGTG
GGCCACAGGGTTGAAATGT

2. Protein encoded by a polynucleotide of claim 1.

3. mRNA transcribed by a of claim 1 and its splicing variants containing at least one of the mutations F66L+V179I, I195N, C82R.

4. Fusion protein comprising the variant of the cytidine deaminase protein encoded by the polynucleotide according to claim
1 and a protein capable of carrying mutants of APOBEC1 on nucleic acids or functional domain thereof.

5. Fusion protein according to claim 4 in the protein capable of carrying APOBEC1 mutants, associated with genomic editing, is chosen from the group consisting of: Zinc-Finger Nucleases (ZFNs), Transcription activator-like effector nucleases (TALENs), Meganucleases, Cas9 and its lab-created variants, Cas12a and its lab-created variants, type SpCas9, VRER SpCas9, VQR SpCas9, EQR SpCas9, xCas9-3.7, eSpCas9 (1.0), eSpCas9 (1.1), Cas9-HF1, HypaCas9, evoCas9, HiFi Cas9, ScCas9, StCas9, NmCas9, SaCas9, CiCas9, CasX), and Cascade/Cas3 and its lab-created variants.

6. Polynucleotide coding for the fusion protein of claims 4 or 5.

7. Vector comprising a polynucleotide according to any one of claims 1 and 3.

8. Host cell comprising a polynucleotide according to any one of claims 1 and 3 and/or expressing a protein encoded by the sequence of claim 2.

9. Use of the fusion protein according to any one of claims 4-5 as a base editor.

10. Base editing process comprising the following stages:
a) cell culture
b) transfection of cells by means of a plasmid vector comprising a sequence coding for the fusion protein of claims 4-5, a sequence coding for a guide RNA and at least one gene for the selection with antibiotic
c) selection of cells that have been transfected with antibiotic until a selected cell culture with the desired genomic mutation is obtained.

11. Kit comprising all the elements necessary to carry out a base editing process by using the fusion protein of claims 4-5 as a base editor comprising at least one plasmid comprising a sequence coding for said fusion protein, a sequence coding for an RNA guide and at least one gene for the selection with antibiotic.

## Patentansprüche

1. Polynukleotid, das für eine Variante des Cytidin-Deaminase-Proteins kodiert, ausgewählt aus der Gruppe bestehend aus:
SEQ.ID.NR.2 
ATGAGTTCCGAGACAGGCCCTGTAGCTGTTGATCCCACTCTGA
GGAGAAGAATTGAGCCCCACGAGTTTGAAGTCTTCTTTGACCC
CCGGGAACTTCGGAAAGAGACCTGTCTGCTGTATGAGATCAAC
TGGGGAGGAAGGCACAGCATCTGGCGACACACGAGCCAAAAC
ACCAACAAACACGTTGAAGTCAATCTCATAGAAAAATTTACTAC
AGAAAGATACTTTTGTCCAAACACCAGATGCTCCATTACCTGGT
TCCTGTCCTGGAGTCCCTGTGGGGAGTGCTCCAGGGCCATTAC
AGAATTTTTGAGCCGATACCCCCATGTAACTCTGTTTATTTATAT
AGCACGGCTTTATCACCACGCAGATCCTCGAAATCGGCAAGGA
CTCAGGGACCTTATTAGCAGCGGTGTTACTATCCAGATCATGA
CGGAGCAAGAGTCTGGCTACTGCTGGAGGAATTTTGTCAACTA
CTCCCCCTCGAATGAAGCTCATTGGCCAAGGTACCCCCATCTG
TGGGTGAGGCTGTACATACTGGAACTCTACTGCATCATTTTAG
GACTTCCACCCTGTTTAAATATTTTAAGAAGAAAACAACCTCAA
CTCACGTTTTTCACGATTGCTCTTCAAAGCTGCCATTACCAAAG
GCTACCACCCCACATCCTGTGGGCCACAGGGTTGAAA
SEQ.ID.NR.3 
ATGAGTTCCGAGACAGGCCCTGTAGCTGTTGATCCCACTCTGA
GGAGAAGAATTGAGCCCCACGAGTTTGAAGTCTTCTTTGACCC
CCGGGAACTTCGGAAAGAGACCTGTCTGCTGTATGAGATCAAC
TGGGGAGGAAGGCACAGCATCTGGCGACACACGAGCCAAAAC
ACCAACAAACACGTTGAAGTCAATTTCATAGAAAAATTTACTACA
GAAAGATACTTTTGTCCAAACACCAGATGCTCCATTACCTGGTT
CCTGTCCTGGAGTCCCTGTGGGGAGTGCTCCAGGGCCATTAC
AGAATTTTTGAGCCGATACCCCCATGTAACTCTGTTTATTTATAT
AGCACGGCTTTATCACCACGCAGATCCTCGAAATCGGCAAGGA
CTCAGGGACCTTATTAGCAGCGGTGTTACTATCCAGATCATGA
CGGAGCAAGAGTCTGGCTACTGCTGGAGGAATTTTGTCAACTA
CTCCCCTTCGAATGAAGCTCATTGGCCAAGGTACCCCCATCTG
TGGGTGAGGCTGTACGTACTGGAACTCTACTGCATCATTTTAG
 
GACTTCCACCCTGTTTAAATAATTTAAGAAGAAAACAACCTCAA
CTCACGTTTTTCACGATTGCTCTTCAAAGCTGCCATTACCAAAG
GCTACCACCCCACATCCTGTGGGCCACAGGGTTGAAA
SEQ.ID.NR.4

ATGAGTTCCGAGACAGGCCCTGTAGCTGTTGATCCCACTCTGA
GGAGAAGAATTGAGCCCCACGAGTTTGAAGTCTTCTTTGACCC
CCGGGAACTTCGGAAAGAGACCTGTCTGCTGTATGAGATCAAC
TGGGGAGGAAGGCACAGCATCTGGCGACACACGAGCCAAAAC
ACCAACAAACACGTTGAAGTCAATtTCATAGAAAAATTTACTACA
GAAAGATACTTTTGTCCAAACACCAGACGCTCCATTACCTGGTT
CCTGTCCTGGAGTCCCTGTGGGGAGTGCTCCAGGGCCATTAC
AGAATTTTTGAGCCGATACCCCCATGTAACTCTGTTTATTTATAT
AGCACGGCTTTATCACCACGCAGATCCTCGAAATCGGCAAGGA
CTCAGGGACCTTATTAGCAGCGGTGTTACTATCCAGATCATGA
CGGAGCAAGAGTCTGGCTACTGCTGGAGGAATTTTGTCAACTA
CTCCCCtTCGAATGAAGCTCATTGGCCAAGGTACCCCCATCTGT
GGGTGAGGCTGTACGTACTGGAACTCTACTGCATCATTTTAGG
ACTTCCACCCTGTTTAAATATTTTAAGAAGAAAACAACCTCAACT
CACGTTTTTCACGATTGCTCTTCAAAGCTGCCATTACCAAAGGC
TACCACCCCACATCCTGTGGGCCACAGGGTTGAAATGT

2. Protein, das durch ein Polynukleotid nach Anspruch 1 kodiert wird.

3. mRNA, die durch ein Polynukleotid nach Anspruch 1 und dessen Spleißvarianten transkribiert wird, enthaltend mindestens eine der Mutationen F66L+V179I, 1195N, C82R.

4. Fusionsprotein, umfassend die Variante des Cytidin-Deaminase-Proteins, welches durch das Polynukleotid gemäß Anspruch 1 kodiert wird, und ein Protein, welches in der Lage ist, Mutanten von APOBEC1 auf Nukleinsäuren oder funktionellen Domänen davon zu tragen.

5. Fusionsprotein, gemäß Anspruch 4, in dem das Protein, das in der Lage ist, APOBEC1-Mutanten zu tragen, die mit Genom-Editing assoziiert sind, aus der Gruppe ausgewählt ist, bestehend aus: Zinkfingernukleasen (ZFNs), transkriptionsaktivatorartigen Effektornukleasen (TALENs), Meganukleasen, Cas9 und seinen im Labor erzeugten Varianten, Cas12a und seinen im Labor erzeugten Varianten, Typ SpCas9, VRER SpCas9, VQR SpCas9, EQR SpCas9, xCas9-7.3, eSpCas9 (1.0), eSpCas (1.1), Cas9-HF1, HypaCas9, evoCas9, HiFi Cas9, ScCas9, StCas9, NmCas9, SaCas9, CiCas9, CasX), und Kaskade/Cas3 und seinen im Labor erzeugten Varianten.

6. Polynukleotid, welches für das Fusionsprotein der Ansprüche 4 oder 5 kodiert.

7. Vektor, der ein Polynukleotid gemäß einem der Ansprüche 1 und 3 umfasst.

8. Wirtszelle, die ein Polynukleotid gemäß einem der Ansprüche 1 und 3 umfasst und/oder ein durch die Sequenz nach Anspruch 2 kodiertes Protein exprimiert.

9. Verwendung des Fusionsproteins gemäß einem der Ansprüche 4-5 als Baseneditor.

10. Verfahren zur Baseneditierung, das die folgenden Phasen umfasst:
a) Zellkultur
b) Transfektion von Zellen mittels eines Plasmidvektors, der eine Sequenz umfasst, welche das Fusionsprotein der Ansprüche 4-5 kodiert, eine Sequenz, die eine Guide-RNA kodiert und mindestens ein Gen für die Selektion mit einem Antibiotikum,
c) Selektion von Zellen, welche mit einem Antibiotikum transfiziert wurden, bis eine selektierte Zellkultur mit der gewünschten genomischen Mutation erhalten wird.

11. Kit, umfassend alle Elemente, die für die Durchführung eines Verfahrens zur Baseneditierung unter Verwendung des Fusionsproteins der Ansprüche 4-5 als Baseneditor notwendig sind, umfassend mindestens ein Plasmid, das eine das genannte Fusionsprotein kodierende Sequenz umfasst, eine RNA-Guide kodierende Sequenz und mindestens ein Gen für die Selektion mit einem Antibiotikum.

## Revendications

1. Polynucléotide codant pour un variant de la protéine cytidine désaminase choisi dans le groupe constitué de :
SEQ.ID.NO. 2 
ATGAGTTCCGAGACAGGCCCTGTAGCTGTTGATCCCACTCTGAGGAGAAGAATTGAGCCCC
ACGAGTTTGAAGTCTTCTTTGACCCCCGGGAACTTCGGAAAGAGACCTGTCTGCTGTATGA
GATCAACTGGGGAGGAAGGCACAGCATCTGGCGACACACGAGCCAAAACACCAACAAACAC
GTTGAAGTCAATCTCATAGAAAAATTTACTACAGAAAGATACTTTTGTCCAAACACCAGAT
GCTCCATTACCTGGTTCCTGTCCTGGAGTCCCTGTGGGGAGTGCTCCAGGGCCATTACAGA
ATTTTTGAGCCGATACCCCCATGTAACTCTGTTTATTTATATAGCACGGCTTTATCACCAC
GCAGATCCTCGAAATCGGCAAGGACTCAGGGACCTTATTAGCAGCGGTGTTACTATCCAGA
TCATGACGGAGCAAGAGTCTGGCTACTGCTGGAGGAATTTTGTCAACTACTCCCCCTCGAA
TGAAGCTCATTGGCCAAGGTACCCCCATCTGTGGGTGAGGCTGTACATACTGGAACTCTAC
TGCATCATTTTAGGACTTCCACCCTGTTTAAATATTTTAAGAAGAAAACAACCTCAACTCA
CGTTTTTCACGATTGCTCTTCAAAGCTGCCATTACCAAAGGCTACCACCCCACATCCTGTG
GGCCACAGGGTTGAAA
SEQ.ID.NO.3 
ATGAGTTCCGAGACAGGCCCTGTAGCTGTTGATCCCACTCTGAGGAGAAGAATTGAGCCCC
ACGAGTTTGAAGTCTTCTTTGACCCCCGGGAACTTCGGAAAGAGACCTGTCTGCTGTATGA
GATCAACTGGGGAGGAAGGCACAGCATCTGGCGACACACGAGCCAAAACACCAACAAACAC
GTTGAAGTCAATTTCATAGAAAAATTTACTACAGAAAGATACTTTTGTCCAAACACCAGAT
GCTCCATTACCTGGTTCCTGTCCTGGAGTCCCTGTGGGGAGTGCTCCAGGGCCATTACAGA
ATTTTTGAGCCGATACCCCCATGTAACTCTGTTTATTTATATAGCACGGCTTTATCACCAC
GCAGATCCTCGAAATCGGCAAGGACTCAGGGACCTTATTAGCAGCGGTGTTACTATCCAGA
TCATGACGGAGCAAGAGTCTGGCTACTGCTGGAGGAATTTTGTCAACTACTCCCCTTCGAA
TGAAGCTCATTGGCCAAGGTACCCCCATCTGTGGGTGAGGCTGTACGTACTGGAACTCTAC
TGCATCATTTTAGGACTTCCACCCTGTTTAAATAATTTAAGAAGAAAACAACCTCAACTCA
CGTTTTTCACGATTGCTCTTCAAAGCTGCCATTACCAAAGGCTACCACCCCACATCCTGTG
GGCCACAGGGTTGAAA
 SEQ. ID. NO. 4

ATGAGTTCCGAGACAGGCCCTGTAGCTGTTGATCCCACTCTGAGGAGAAGAATTGAGCCCC
ACGAGTTTGAAGTCTTCTTTGACCCCCGGGAACTTCGGAAAGAGACCTGTCTGCTGTATGA
GATCAACTGGGGAGGAAGGCACAGCATCTGGCGACACACGAGCCAAAACACCAACAAACAC
GTTGAAGTCAATtTCATAGAAAAATTTACTACAGAAAGATACTTTTGTCCAAACACCAGAC
GCTCCATTACCTGGTTCCTGTCCTGGAGTCCCTGTGGGGAGTGCTCCAGGGCCATTACAGA
ATTTTTGAGCCGATACCCCCATGTAACTCTGTTTATTTATATAGCACGGCTTTATCACCAC
GCAGATCCTCGAAATCGGCAAGGACTCAGGGACCTTATTAGCAGCGGTGTTACTATCCAGA
TCATGACGGAGCAAGAGTCTGGCTACTGCTGGAGGAATTTTGTCAACTACTCCCCtTCGAA
TGAAGCTCATTGGCCAAGGTACCCCCATCTGTGGGTGAGGCTGTACGTACTGGAACTCTAC
TGCATCATTTTAGGACTTCCACCCTGTTTAAATATTTTAAGAAGAAAACAACCTCAACTCA
CGTTTTTCACGATTGCTCTTCAAAGCTGCCATTACCAAAGGCTACCACCCCACATCCTGTG
GGCCACAGGGTTGAAATGT

2. Protéine codée par un polynucléotide selon la revendication 1.

3. ARNm transcrit par un polynucléotide selon la revendication 1 et ses variants d'épissage contenant au moins une des mutations F66L+V179I, I195N, C82R.

4. Protéine de fusion comprenant le variant de la protéine cytidine désaminase codé par le polynucléotide selon la revendication 1 et une protéine capable de transporter des mutants d'APOBEC1 sur des acides nucléiques ou un domaine fonctionnel de ceux-ci.

5. Protéine de fusion selon la revendication 4, dans la protéine capable de transporter des mutants d'APOBEC1, associée à une édition génomique, est choisie dans le groupe constitué : des nucléases à doigt de zinc (ZFN), des nucléases effectrices de type activateur de transcription (TALEN), des méganucléases, de Cas9 et de ses variants créés en laboratoire, de Cas12a et de ses variants créés en laboratoire, type SpCas9, VRER SpCas9, VQR SpCas9, EQR SpCas9, xCas9-3.7, eSpCas9 (1.0), eSpCas9 (1.1), Cas9-HF1, HypaCas9, evoCas9, HiFi Cas9, ScCas9, StCas9, NmCas9, SaCas9, CiCas9, CasX), et de Cascade/Cas3 et de ses variants créés en laboratoire.

6. Polynucléotide codant pour la protéine de fusion selon les revendications 4 ou 5.

7. Vecteur comprenant un polynucléotide selon l'une quelconque des revendications 1 et 3.

8. Cellule hôte comprenant un polynucléotide selon l'une quelconque des revendications 1 et 3 et/ou exprimant une protéine codée par la séquence selon la revendication 2.

9. Utilisation de la protéine de fusion selon l'une quelconque des revendications 4-5 comme éditeur de base.

10. Procédé d'édition de base comprenant les étapes suivantes :
a) une culture de cellules
b) la transfection des cellules au moyen d'un vecteur plasmidique comprenant une séquence codant pour la protéine de fusion selon les revendications 4-5, une séquence codant pour un ARN guide et au moins un gène pour la sélection avec un antibiotique
c) la sélection des cellules qui ont été transfectées avec un antibiotique jusqu'à l'obtention d'une culture de cellules sélectionnées ayant la mutation génomique souhaitée.

11. Kit comprenant tous les éléments nécessaires pour effectuer un procédé d'édition de base en utilisant la protéine de fusion selon les revendications 4-5 comme éditeur de base comprenant au moins un plasmide comprenant une séquence codant pour ladite protéine de fusion, une séquence codant pour un ARN guide et au moins un gène pour la sélection avec un antibiotique.
